# EUROPÄISCHE PATENTANMELDUNG

(11) **EP 4 462 379 A1**
(43) Veröffentlichungstag der Anmeldung: **13.11.2024**
(21) Anmeldenummer: 24172003.6
(22) Anmeldetag: 23.04.2024
(51) Int. Cl.: G06V 10/774

(54) **VERFAHREN ZUR ERZEUGUNG SYNTHETISCHER DATEN ZUM KI-MODELLTRAINING**

(30) Priorität: 10.05.2023 DE 102023112359
(71) Anmelder: Karl Storz SE & Co. KG, 78532 Tuttlingen (DE)
(72) Erfinder: Khazatskiy, Igor, 78532 Tuttlingen (DE); Aderhold, Andrej, 78532 Tuttlingen (DE); Balashov, Alexander, 78532 Tuttlingen (DE)

(57) **Zusammenfassung**

Computerimplementiertes Verfahren zum Erzeugen anwendungsspezifischer Daten zum Trainieren einer auf künstlicher Intelligenz basierender Objekterkennung umfassend das Erzeugen einer Befehlsdatei zum Ausführen auf einem Prozessor zur Erzeugung Bilddatensatzes unter Verwendung wenigstens einer Konfigurationsdatei, das Erzeugen wenigstens einer Konfigurationsdatei die eine zu simulierende Szene beschreibt, das Übertragen und Ausführen der Befehlsdatei und der Konfigurationsdatei auf den Prozessor zum Erzeugen des Bilddatensatzes und einer mit dem Bilddatensatz assoziierten Annotationsdatei und das Speichern des Bilddatensatzes zusammen mit der Annotationsdatei.

## Beschreibung

### Technisches Gebiet

Die vorliegende Erfindung betrifft Verfahren zum Erzeugen synthetischer Trainingsdaten für das Trainieren auf künstliche Intelligenz basierender Modelle.

### Hintergrund

Maschinelles Lernen ist der Bereich der künstlichen Intelligenz, der sich mit dem selbstständigen Erschließen von Zusammenhängen auf Basis von Beispieldaten beschäftigt. Hierbei werden Algorithmen mit strukturierten Daten "gefüttert", diese lernen also aus den Daten und können basierend darauf Vorhersagen treffen. Die Algorithmen funktionieren, indem sie ein mathematisches Modell aus Eingabedaten erstellen, aufgrund dessen sie dann datengesteuerte Vorhersagen oder Entscheidungen treffen können.

Insbesondere überwachte Lernalgorithmen können vielversprechende Lösungen für viele reale Probleme, wie Textklassifizierung, Objektklassifizierung und -erkennung, medizinische Diagnose und Informationssicherheit bieten. Damit überwachtes Lernen funktioniert, wird ein Datensatz benötigt, aus dem das Modell lernen kann, richtige Entscheidungen zu treffen.

Eine große Einschränkung des überwachten Lernens in realen Anwendungen ist die Schwierigkeit, Daten zum Trainieren der Vorhersagemodelle zu erhalten. Es ist bekannt, dass die Klassifikationsleistung eines Vorhersagemodells entscheidend von der Qualität der Trainingsdaten abhängt. Idealerweise möchte man Klassifikatoren mit diversen, strukturierten Daten trainieren, die alle Klassen vollständig repräsentieren, was neben der Auswahl der geeigneten Daten auch einen entsprechenden Umfang erfordert.

Es gibt jedoch Fälle, in denen die zu lösende Aufgabe einen Nischenbereich mit geringer Datenlage betrifft, oder aus anderen, z.B. regulatorischen Gründen die Verfügbarkeit von Daten beschränkt ist, und die Beschaffung des richtigen Datensatzes an sich eine Herausforderung darstellt. Beispiele hierfür sind insbesondere Verfahren zur Objekterkennung im medizinischen und/oder klinischen Bereich, zum Beispiel in der Überwachung oder Unterstützung von operativen Eingriffen, bei denen nicht zuletzt aufgrund von Datenschutzerfordernissen wenig bis keine Daten verfügbar sind.

Wenn jedoch für ein bestimmtes Szenario nicht ausreichend Daten zur Verfügung stehen, sind die Vorhersagen des Modells möglicherweise nicht genau oder verzerrt. Es gibt Möglichkeiten wie Datenerweiterung und Datenkennzeichnung, die helfen können, die Mängel zu beheben, aber das Ergebnis ist möglicherweise immer noch nicht genau oder nicht zuverlässig.

Entsprechend können beispielsweise basierend auf echten Daten, wie zum Beispiel Bilddaten, Variationen durch systematische Veränderungen erzeugt werden und so der Datensatz erweitert werden. Daneben sind im Stand der Technik auch Ansätze zur Erzeugung synthetischer Daten bekannt. Problematisch ist hierbei oft die mangelnde Diversität der Daten, was zu ungenauen Vorhersagemodellen führen kann.

Die Datenkennzeichnung ist der Prozess der Identifizierung von Rohdaten (Bilder, Textdateien, Videos usw.) und das Hinzufügen einer oder mehrerer aussagekräftiger und informativer Kennzeichnungen (Label), um Kontext bereitzustellen, damit ein maschinelles Lernmodell daraus lernen kann. Solche Labels könnten beispielsweise angeben, ob ein Foto einen Vogel oder ein Auto enthält, welche Wörter in einer Audioaufnahme geäußert wurden, oder ob ein Röntgenbild einen Tumor enthält.

Die Datenkennzeichnung beginnt normalerweise damit, dass Menschen entsprechende Urteile zu unstrukturierten Daten fällen, und diese so strukturieren. Die Kennzeichnung kann so grob sein wie ein einfaches Ja/Nein oder so granular wie das Identifizieren der spezifischen Pixel in einem Bild, die einem bestimmten Objekt zugeordnet sind. Das Kennzeichnen von Rohdaten ist entsprechend - je nach erforderlicher Granularität und Qualität - beliebig aufwendig und kostenintensiv.

Das maschinelle Lernmodell verwendet diese Kennzeichnungen, um die zugrunde liegenden Muster zu lernen. Das Ergebnis ist ein trainiertes Modell, das verwendet werden kann, um Vorhersagen zu neuen Daten zu treffen. Die Genauigkeit Ihres trainierten Modells hängt von der Genauigkeit des gekennzeichneten Datensatzes ab. Wenn nicht ausreichend Daten mit entsprechender Kennzeichnung zur Verfügung stehen, ist die Qualität und damit die Anwendbarkeit des trainierten Modells beschränkt und die von dem Modell getroffenen Aussagen gegebenenfalls nicht verlässlich.

Entsprechend ist es eine Aufgabe der vorliegenden Erfindung die Einschränkungen oder Defizite der aus dem Stand der Technik bekannten Verfahren, wenigstens teilweise, zu überwinden.

### Zusammenfassung der Erfindung

Aufgabe der vorliegenden Erfindung ist es, die im Stand der Technik bekannten Nachteile, wenigstens teilweise, zu überwinden. Die vorstehende Aufgabe wird durch erfindungsgemäßes Verfahren gemäß den Ansprüchen 1, 13 und 14 gelöst. Bevorzugte Ausgestaltungsformen der Erfindung sind Gegenstand der entsprechenden Unteransprüche.

Entsprechend offenbart die vorliegende Erfindung ein computerimplementiertes Verfahren zum Erzeugen anwendungsspezifischer Daten zum Trainieren einer auf künstlicher Intelligenz basierender Objekterkennung in Bildern medizinischer und/oder klinischer Arbeitsabläufe, umfassend das Erzeugen einer Befehlsdatei zum Ausführen auf einem Prozessor zur Erzeugung eines bis zu dreidimensionalen Bilddatensatzes unter Verwendung wenigstens einer Konfigurationsdatei, das Erzeugen wenigstens einer Konfigurationsdatei die eine zu simulierende Szene beschreibt, wobei die Konfiguration wenigstens eine Kamera und wenigstens eine Lichtquelle beschreibt, das Übertragen und Ausführen der Befehlsdatei und der Konfigurationsdatei auf den Prozessor zum Erzeugen des Bilddatensatzes, wobei der Bilddatensatz perspektivisch der wenigstens einen Kamera entspricht und die wenigstens eine Lichtquelle berücksichtigt, das Erzeugen einer mit dem Bilddatensatz assoziierten Annotationsdatei und das Speichern des Bilddatensatzes zusammen mit der Annotationsdatei.

In bevorzugten Ausführungsformen des erfindungsgemäßen Verfahrens, kann die Konfigurationsdatei eine statische oder dynamische Szene beschreiben.

In bevorzugten Ausführungsformen des erfindungsgemäßen Verfahrens kann die Konfigurationsdatei allgemeine Parameter, Objekte, Materialien, Beleuchtung, Kameraeigenschaften, Objektpositionen, Objektbewegungen Umgebungseigenschaften, Okklusionsebenen und/oder zeitabhängige Änderungen von Parametern umfassen. Optional kann die Konfigurationsdatei auf Objekten und/oder Materialien in einem nicht flüchtigen Speicher verweisen, die bei dem Ausführen der Befehlsdatei zum Erzeugen des Bilddatensatzes hinzugefügt werden. Diese Objekte können in einer entsprechenden Datenbank hinterlegt werden und in dieser Form als abrufbare Beschreibungen und/oder Bildinformationen in die Erzeugung der Szene eingebunden werden.

In bevorzugten Ausführungsformen des erfindungsgemäßen Verfahrens kann die in der Konfigurationsdatei beschriebene Szene, die beispielsweise einen medizinischen und/oder klinischen Arbeitsablauf darstellen soll, in einer vorbestimmten Umgebung zu simulieren sein, wobei die vorbestimmte Umgebung einen realen Raum beschreibt. Arbeitsabläufe können hierbei bestimmte medizinische Eingriffe oder auch bestimmte Abläufe in einem bestimmten Bereich eines Klinikums, wie eine bestimmte Operation in einem OP-Saal oder auch die Vorbereitung oder auch Reinigung eines OP-Saals sein. Die vorbestimmte Umgebung kann in Form von Beschreibung und/oder Bilddaten in einer Datenbank hinterlegt werden. Beispielsweise kann eine detaillierte Beschreibung eines Operationssaals mit genauen Größeninformationen und gegebenenfalls mit entsprechenden Fotos in einer Datenbank hinterlegt sein. Der Benutzer kann die entsprechende Umgebung, wie beispielsweise einen bestimmten Operationssaal bei der Szenenbeschreibung auswählen damit die generierte Szene dann in dieser ausgewählten Umgebung abläuft. Hierbei kann in Ausführungsformen des Verfahrens die Beschreibung der Umgebung beziehungsweise des realen Raums über eine Scanvorrichtung, wie einen LIDAR-scanner erfasst werden und/oder aus digitalen Bildern konstruiert werden. Insbesondere können diese Informationen auch Informationen über vorhandene (Raum-)Kameras enthalten. Den mit dem Verfahren erzeugten Szenen liegen dann die Umgebungsinformationen als feste Parameter zugrunde, wodurch die Spezifität der Daten für das Training von Objekterkennungsalgorithmen in dem speziellen Umfeld erheblich gesteigert werden kann.

In bevorzugten Ausführungsformen des erfindungsgemäßen Verfahrens können die allgemeinen Parameter eine Beschreibung der zu simulierenden Szene umfassen. Hierbei können insbesondere auch bestimmte Klassen von zu simulierenden Szenen bestimmt und durch den Benutzer ausgewählt werden. So können beispielsweise - am Beispiel des Operationssaales bleibend - bestimmte Arten von Operationen (beispielsweise ausgereichtet an den zu operierenden Bereich des Körpers) mit jeweiligen spezifischen, wiederkehrenden Eigenschaften (beispielsweise bezogen auf die Position des Operateurs) bestimmt werden.

In bevorzugten Ausführungsformen des erfindungsgemäßen Verfahrens können basierend auf Variationen der in der Konfigurationsdatei enthaltenen Beschreibungen weitere Bilddatensätze und mit den weiteren Bilddatensätzen assoziierte Annotationsdateien erzeugt werden. Hierbei kann insbesondere in der Konfigurationsdatei festgelegt sein, welche Variationen durchgeführt werden sollen, beziehungsweise welche Variationen möglich sind. So kann in Ausführungsformen des Verfahrens die Konfigurationsdatei erlaubte und verbotene Zustände von den Objekten und der Beleuchtung enthalten und die Variationen durch diese Zustände definiert werden. Entsprechend können die Variationen Änderungen der Lichtquelle(n) beziehungsweise der Beleuchtung und/oder der Schattenbildung enthalten. Alternativ oder in Ergänzung dazu können die Variationen die Anwesenheit, Position und/oder Bewegung verschiedener Objekte betreffen.

In bevorzugten Ausführungsformen des erfindungsgemäßen Verfahrens können die erzeugten Bilddatensätze, und die mit ihnen assoziierten Annotationsdateien, kategorisiert gespeichert werden.

In bevorzugten Ausführungsformen des erfindungsgemäßen Verfahrens kann die Konfigurationsdatei wenigstens teilweise Informationen basierend auf einer Voreinstellung enthalten. Voreinstellungen können insbesondere allgemeine, wiederkehrende Eigenschaften einer zu simulierenden Szene enthalten. Alternativ oder in Ergänzung dazu können die Voreinstellungen auch bestimmte Ausgestaltungen und/oder Eigenschaften von Objekten und/oder Materialine spezifizieren und so die beispielsweise die möglichen Variationen oder die Auswahl aus Objekten und/oder Materialien aus der Datenbank beschränken. So kann beispielsweise durch eine ein bestimmtes Krankenhaus bestimmende Voreinstellung die Farbe der Arbeitskleidung des Klinikpersonals vorausgewählt werden. Auf diese Weise können die generierten Bilddaten weiter spezifiziert werden und - über Verwendung als Trainingsdaten für eine Objekterkennung - die resultierende Objekterkennung verbessert werden.

In einem weiteren Aspekt ist die Erfindung auf ein Verfahren zum Erzeugen einer anwendungsspezifischen, auf künstlicher Intelligenz basierender Objekterkennung, umfassend das Trainieren eines Vorhersagealgorithmus mit nach dem vorstehend beschriebenen Verfahren erstellten Trainingsdaten.

In einem weiteren Aspekt ist die Erfindung auf ein Verfahren zum Erkennen von Objekten in einer spezifischen Anwendung, wobei ein mit nach einem der vorstehend beschriebenen Verfahren erstellten Trainingsdaten trainierter Vorhersagealgorithmus auf wenigstens ein digitales Bild angewendet wird und wobei das wenigstens eine Bild, ein Bild einer Szene ist oder eine sonstigen, gegebenenfalls über Voreinstellungen in der Konfigurationsdatei bestimmten Eigenschaft aufweist, die Grundlage der erstellten Trainingsdaten ist.

In bevorzugten Ausführungsformen des erfindungsgemäßen Verfahrens zum Erkennen von Objekten in einer spezifischen Anwendung wird der spezifisch trainierte Vorhersagealgorithmus basierend auf der Erkennung der spezifischen Anwendung ausgewählt. Hierbei kann beispielsweise durch Auslesen einer Raumkamera ein bestimmter Raum erkannt werden und den entsprechenden, auf diesen Raum trainierte Algorithmus ausgewählt werden. Alternativ oder in Ergänzung dazu können anderen Informationen, beispielsweise Operationssaal-Belegungspläne zugrunde gelegt werden, um die Art des Eingriffs zu erfassen und sie den entsprechenden, auf diesen Eingriff trainierte Algorithmus auszuwählen. Hierbei sind je nach Spezifikation der erfindungsgemäß erzeugten Trainingsdaten alle hierbei zugrunde gelegten Parameter für die Auswahl spezifischer Algorithmen möglich.

### Kurzbeschreibung der Abbildungen

Abbildung 1 zeigt eine schematische Darstellung des Verfahrens.

Abbildungen 2a und 2b zeigen erzeugte Bilddaten.

Die beiliegenden Zeichnungen veranschaulichen beispielhafte Ausführungsformen der Erfindung und dienen der beispielhaften Erläuterung der Grundsätze der Erfindung.

### Detaillierte Beschreibung

Die vorliegende Erfindung wird nun unter Bezugnahme auf die beiliegenden Zeichnungen näher beschrieben. Die Erfindung kann jedoch in vielen verschiedenen Formen verkörpert werden und sollte nicht auf die hier dargelegten Ausführungsformen beschränkt ausgelegt werden. Es sei angemerkt, dass die Abbildungen allgemeine Merkmale der in den jeweiligen Ausführungsformen genutzten Verfahren veranschaulichen. Diese Abbildungen geben jedoch die exakte Struktur oder das exakte Merkmal einer gegebenen Ausführungsform möglicherweise nicht exakt wieder. Zudem bezeichnen gleiche Bezugszeichen in den Abbildungen entsprechende Teile über die verschiedenen Ansichten oder Ausführungsformen hinweg.

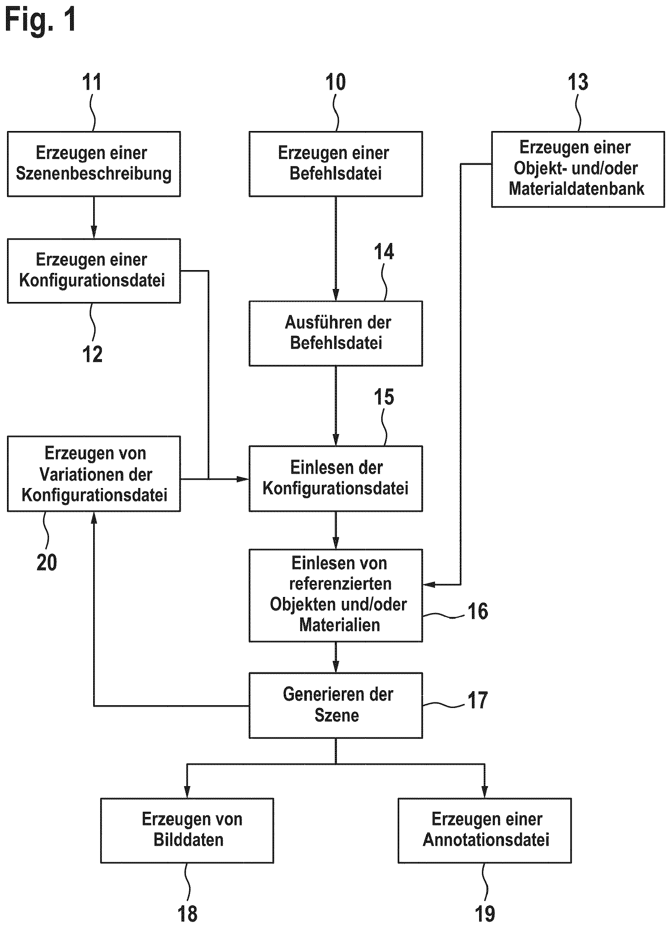

Abbildung 1 zeigt hierbei eine schematische Darstellung des erfindungsgemäßen Verfahrens. In einem ersten Schritt 10 wird eine Befehlsdatei zum Ausführen auf einem Prozessor zur Erzeugung eines bis zu dreidimensionalen Bilddatensatzes unter Verwendung wenigstens einer Konfigurationsdatei. Die Befehlsdatei kann je nach Anwendung im Vorfeld erzeugt werden und für verschiedene Durchführungen des erfindungsgemäßen Verfahrens verwendet werden. Für die Erzeugung des Bilddatensatzes durch den Prozessor wird vorzugsweise ein entsprechendes auf dem Prozessor ausführbares Computerprogramm, insbesondere ein Computerprogramm zur Erstellung sogenannter "computer generated imagery" (CGl) wie beispielsweise Blender, verwendet. Entsprechend ist die in der Befehlsdatei zu verwendende Befehlssyntax abhängig davon, welche Befehlssyntax der Prozessor in Verbindung mit dem Computerprogramm verarbeiten kann. So kann eine Befehlsdatei, wie in dem nachfolgenden Beispielcode, beispielsweise auf Python^{™} basieren.

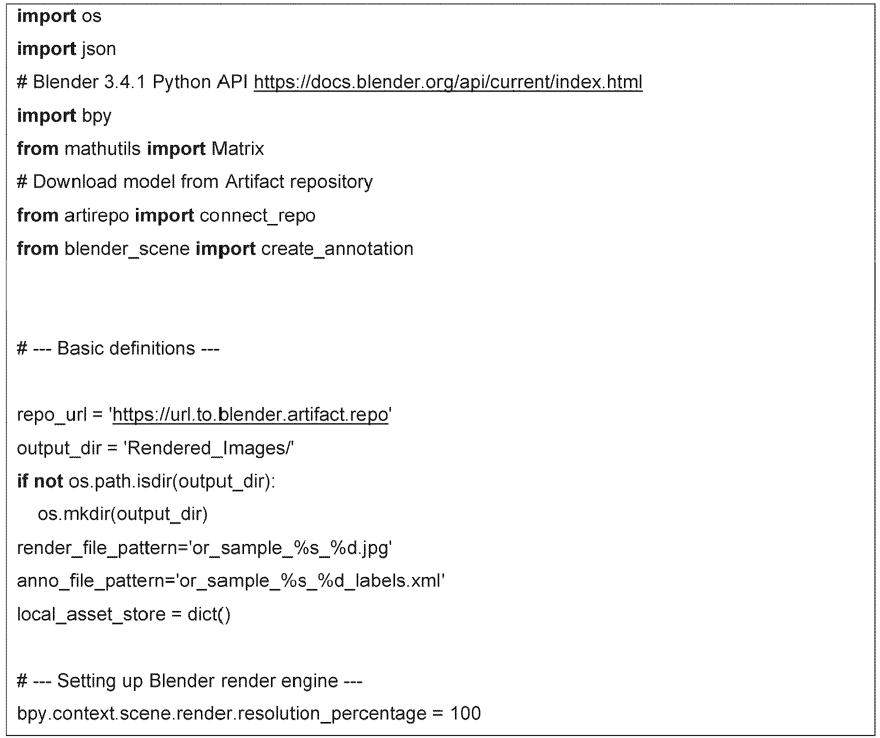

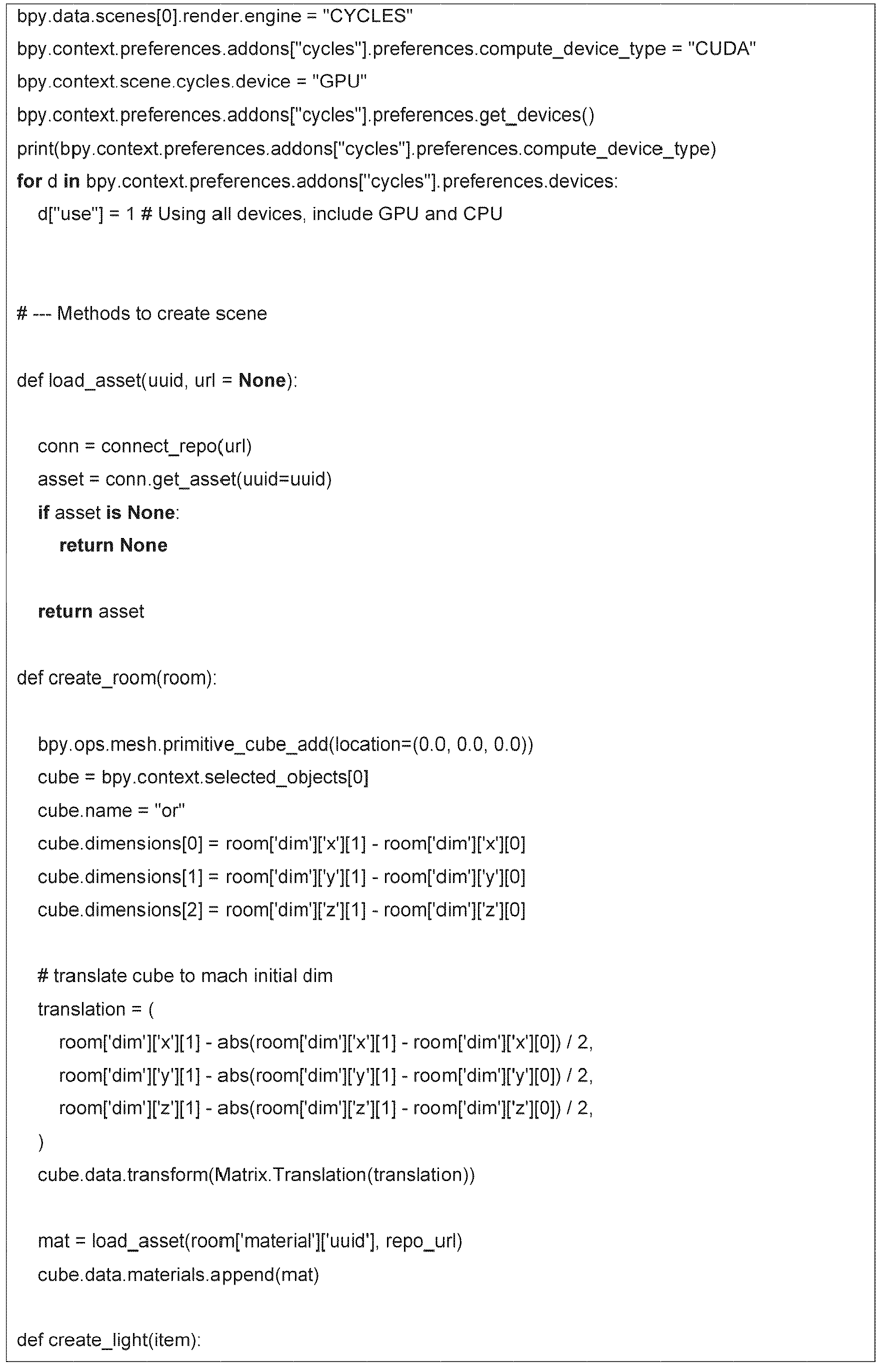

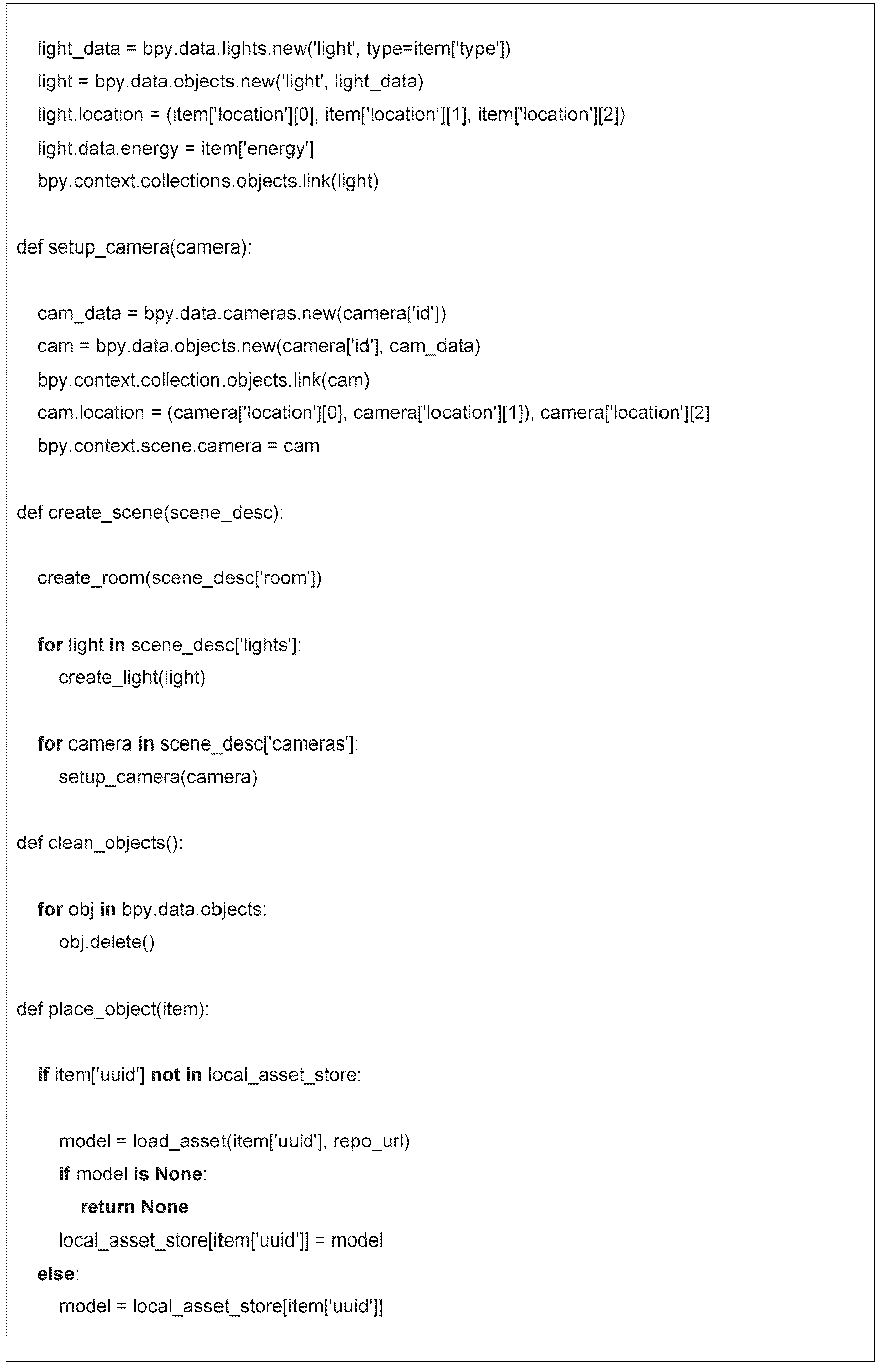

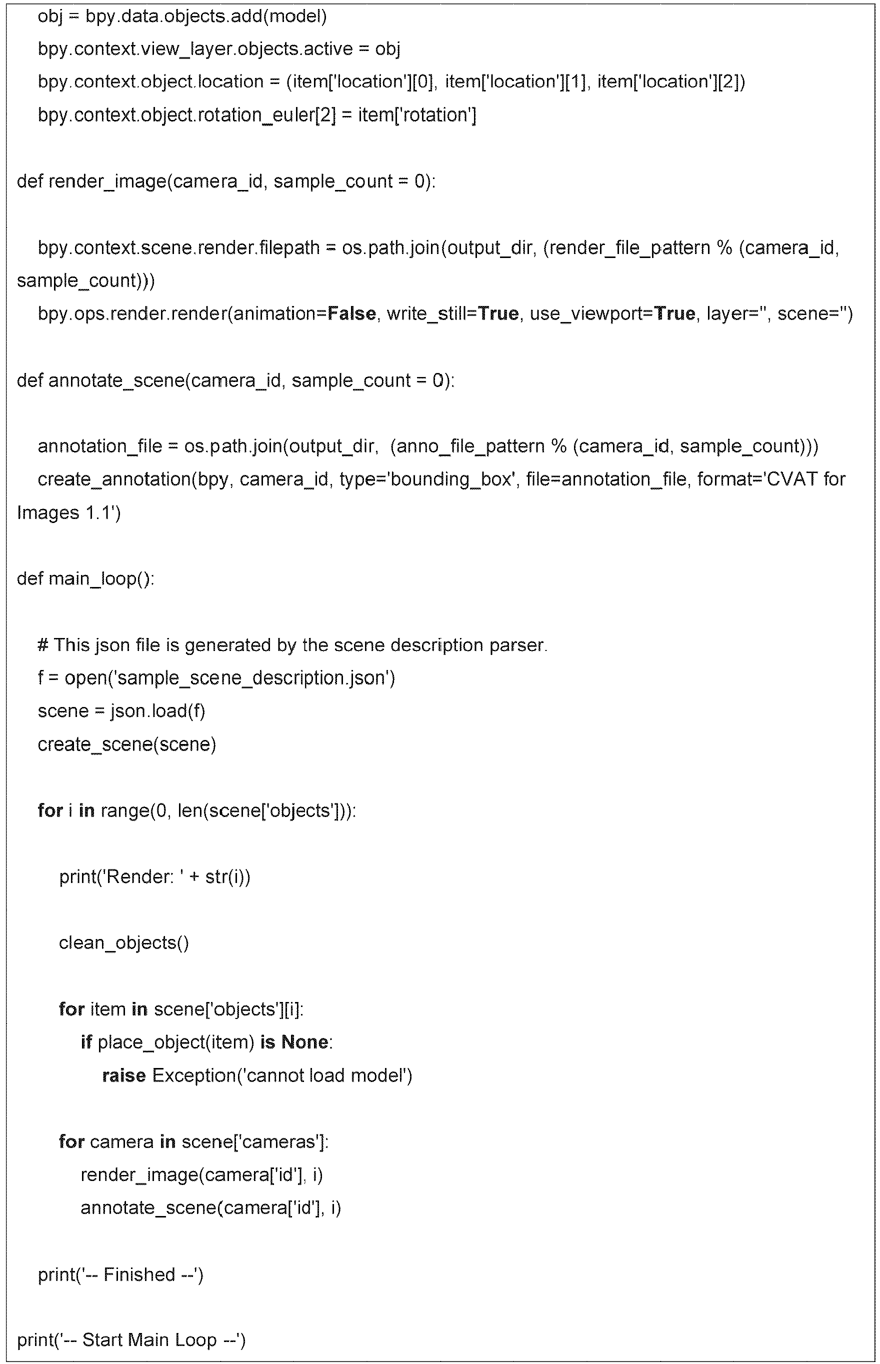

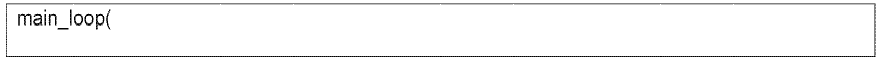

Beispielcode einer Befehlsdatei "sample_blnder_api_render.py"

Die Befehlsdatei verweist auf eine Konfigurationsdatei (wie nachfolgend ausgeführt), die alle für die zu generierende Szene relevanten Informationen enthält und kann somit sehr generisch gehalten und somit für verschiedene Szene verwendetet werden.

Die vorstehende Beispielcode der Befehlsdatei ist auf die Ausführung auf einem Prozessor unter der Verwendung der 3D-Grafiksuite "Blender" ausgelegt und benutzt eine entsprechende Blender API (bpy) die selbst Funktionen des Programms Blender ausführen kann. Der Code verweist hierbei auf die Konfigurationsdatei, in diesem Fall "sample_scene_description. json", und verarbeitet so die, wie nachfolgend beschrieben, von dem Benutzer eingegebenen Voreinstellungen für die zu generierende Szene.

In einem weiteren Schritt 11 des erfindungsgemäßen Verfahrens erstellt ein Benutzer eine vorzugsweise digitale Beschreibung des gesamten Inhalts einer in einer geschlossenen Umgebung zu simulierenden Szene. Für diese Beschreibung können sowohl vordefinierte Elemente und/oder Parameter sowie durch den Benutzer bestimmte Elemente und/oder Parameter verwendet werden. Vordefinierte Elemente und/oder Parameter, wie Umgebungsbeschreibungen, Objekte, Personen, gegebenenfalls mit dezidierten Rollen, Materialien und dergleichen können in einer entsprechenden Datenbank hinterlegt, und so dem Benutzer zur Verfügung gestellt werden. Die vordefinierten Elemente können als Beschreibung, als Bilddaten und/oder als Kombinationen von Beschreibung und Bilddaten vorliegen. Bilddaten können sowohl als digitale Bilder realer Elemente, gerendert oder als Kombination von beidem vorliegen. In der Beschreibung werden alle für die zu simulierende Szene relevanten Parameter, Objekte, Beleuchtung, Kameraeigenschaften, Objektpositionen, Umgebungseigenschaften, Okklusionsebenen und/oder zeitabhängige Änderungen von Parametern und Bewegungen von Objekten beschrieben. Die simulierten Bewegungen führen zu einer dynamischen Szene, wobei die Bilddatensätze bevorzugt einen aus entsprechenden Einzelbildern bestehenden Videostream darstellen.

Entsprechend kann beispielsweise die Umgebung, in der die Szene ablaufen soll, beschrieben werden, oder eine entsprechende vordefinierte Beschreibung der Umgebung, wie die Beschreibung eines Operationssaals, vom Benutzer ausgewählt werden. Die Beschreibung der Umgebung kann hier die Dimensionen, gegebenenfalls Fenster und/oder Türen, Lichtquellen und die Position wenigstens einer Kamera aus deren Blickwinkel die Darstellung der simulierten Szene erfolgen soll, Kameraauflösung und Bildqualität, Eigenschaften der in der Umgebung verwendeten Materialen, wie beispielsweise Wand und Bodenmaterialien umfassen. Die Beschreibung der Umgebung kann hier als vordefinierte Beschreibung zur Auswahl durch den Benutzer in der entsprechenden Datenbank hinterlegt sein, wobei diese vordefinierte Beschreibung die Beschreibung eines virtuellen Raums oder eines real existierenden Raums sein kann. Im Fall eines real existierenden Raums kann die Beschreibung auch Informationen die durch eine entsprechende Vorrichtung zur dreidimensionalen Erfassung des Raums, wie beispielsweise mittels dreidimensionalem Laserscanning oder dergleichen erfolgen. insbesondere können auch digitale Bilder eines real existierenden Raums, bevorzugt in Kombination mit entsprechenden Tiefeninformationen, erfasst werden. Solche digitalen Bilder können insbesondere auch als Teil oder Grundlage der nachfolgenden Simulation der Szene verwendet werden. Des Weiteren können auch Kameraeigenschaften, optische Fehler oder Störungen des optischen Systems, mit dem die digitalen Bilder der realen Umgebung erfasst wurden, extrahiert und als digitaler Filter verwendet werden. Diese digitalen Filter können im Simulationsprozess auf das gerenderte Bildmaterial angewandt werden.

Vordefinierte Beschreibungen können durch den Benutzer verändert, erweitert, erstellt und auch für die weitere Verwendung gespeichert werden.

So kann beispielsweise ein bestimmter Operationssaal in einem Krankenhaus vermessen und in Form einer vordefinierten Beschreibung zur weiteren Verwendung gespeichert werden. Die vordefinierte Beschreibung des Operationssaals kann neben der reinen Dimensionierung auch beliebige weitere Objekte, wie beispielsweise OP-Einrichtungsgegenstände, Beleuchtung, Raumkameras, festinstallierte Geräte und dergleichen umfassen. Die vordefinierte Beschreibung kann darüber hinaus, wie vorstehend ausgeführt, auch digitale Bilder, insbesondere mit zusätzlichen Tiefeninformationen, enthalten, die teilweise oder als Grundlage für die nachfolgende Simulation der Szene verwendet werden können.

Nach Auswahl der Umgebung für die Simulation können durch den Benutzer weitere Elemente für die Simulation aus der Datenbank ausgewählt. oder direkt beschrieben werden. Beschreibungen einzelner Elemente für die Simulation können ebenfalls durch den Benutzer verändert, erweitert, erstellt und auch für die weitere Verwendung gespeichert werden. So können bestimmte in der zu simulierenden Szene auftretende Objekte und Geräte und auch Personen hinzugefügt werden. Im Beispiel des Operationssaals können Personen wie beispielsweise Patienten, Reinigungspersonal, operationstechnische/r Assistent/in und/oder Ärzte hinzugefügt werden. Hierbei können in der Beschreibung auch krankenhausspezifische Aspekte wie die Kleidungsfarbe berücksichtigt werden. Des Weiteren können für die Operation benötigte Gerätschaften hinzugefügt und gegebenenfalls detaillierter beschrieben werden. Darüber hinaus kann auch beschrieben werden, welche Objekte sich in welcher Form in der Simulation bewegen sollen oder dürfen; die Bewegungen können entweder konkret definiert werden, oder in der nachfolgenden Simulation automatisch und - bevorzugt innerhalb bestimmter Vorgaben - zufällig ausgeführt werden.

Gleichermaßen können umfassendere Szenenbeschreibungen als eine vordefinierte Beschreibung zur weiteren Verwendung gespeichert, und für die Erstellung neuer Simulationen verwendet werden. So kann - wiederum im Beispiel des Operationssaals - die instrumentelle und/oder personelle Ausstattung für einen bestimmten Eingriff, wie beispielsweise eine Laparoskopie ausgewählt, und durch den Benutzer gegebenenfalls angepasst werden.

Daneben können allgemeine Parameter für die Simulation definiert werden. So können - sofern nicht bereits in einer Beschreibung enthalten - Art und Ort der Beleuchtung und der Kamera ausgewählt werden. Wenn die Simulation auf Grundlage einer realen Umgebung, wie einem realen Operationssaal, erfolgen soll, können Beleuchtung und Kamera(s) der Situation in der realen Umgebung angepasst werden. Hierbei können auch individuelle Charakteristika der realen Elemente, die in der Simulation nachgebildet werden sollen, aufgenommen werden. So können beispielsweise die Eigenschaften der realen Kamera berücksichtigt werden. Wenn nachfolgend ein mit den Simulationsdaten trainiertes Vorhersagemodell in eben dieser Umgebung, basierend auf mit der realen Kamera erfassten Bildern, angewendet werden soll, kann durch vorherige Berücksichtigung der Kameraeigenschaften die Qualität der Vorhersagen gesteigert werden.

Die einzelnen in der Konfigurationsdatei aufgeführten oder durch den Benutzer eingeführten oder ausgewählten Elemente sind vorzugsweise mit erlaubten und verbotenen Zuständen beschrieben. Entsprechend können bestimmte Objekte als unbeweglich, andere als beweglich und unbeweglich beschrieben werden. So kann ein Krankenbett als beweglich, jedoch immer auf dem Boden stehend, innerhalb bestimmter Grenzen in der Höhe veränderbar definiert werden, wohingegen ein Operationstisch als stationär, auf dem Boden stehend und in der Höhe veränderbar definiert werden kann. Bestimmten Personengruppen können in Abhängigkeit ihrer Rollen bestimmte Bewegungs-, beziehungsweise Aufenthaltsbereiche zugeordnet werden. So kann beispielsweise im Beispiel des Operationssaals ein Patient auf einem Krankenbett oder auf dem Operationstisch liegen, wohingegen Krankenhauspersonal weder auf Bett oder Tisch liegen kann, aber sonst im Operationssaal in der Simulation der Szene frei angeordnet und bewegt werden kann. Die Definition erlaubter und verbotener Zustände kann beliebig ausführlich sein; so können beispielsweise für einen bestimmten medizinischen Eingriff bestimmte Gerätschaften oder Personen spezifische mögliche Positionen zugeordnet werden. So unterscheiden sich beispielsweise die Positionen von OP-Personal und Gerätschaften bei HNO-Operationen gegenüber der Position bei Eingriffen an den Füßen signifikant. Die Definitionen der Zustände können auch in sich eine Hierarchie aufweisen, das heißt, dass ein Objekt A auf einem Objekt B angeordnet sein kann, nicht aber umgekehrt. Entsprechend können auch Definitionen von Okklusionsebenen hierarchische Informationen umfassen. Der Benutzer hat an dieser Stelle auch die Möglichkeit, einzelne Elemente in der Beschreibung als konstant zu bestimmen, das heißt, der einzig erlaubte Zustand für dieses Element ist der vom Benutzer in der Beschreibung der Szene initial bestimmte Zustand. Manche vordefiniert Elemente, die der Benutzer der Beschreibung hinzufügt, wie beispielsweise Wände oder fest installiertes Mobiliar oder Gerätschaften, sind grundsätzlich als konstant definiert. Durch die Definition von erlaubten und verbotenen Zuständen können die Simulationen realistischer gestaltet werden, da keine in der Realität ausgeschlossenen Szenen erzeugt werden. Gleichermaßen können anwendungsspezifische Zustandsdefinitionen, wie beispielsweise die vorstehend erwähnte Unterscheidung zwischen einer HNO-Operation und einer Fußoperation, zu spezifischen Vorhersagemodellen mit entsprechend hoher Vorhersagequalität für die jeweilige Anwendung führen.

Unter Verwendung eines geeigneten Computerprogramms (Parser), werden in einem weiteren Schritt 12 des erfindungsgemäßen Verfahrens die Eingaben des Benutzers in ein für die Weiterverarbeitung geeigneteres Format überführt und so die Konfigurationsdatei, auf die in der Befehlsdatei verweisen wird, erzeugt. Die hierbei möglichen Formate sind abhängig von dem nachfolgend eingesetzten Computerprogramm; ein mögliches und in dem nachfolgenden Beispielcode verwendetes Format ist JavaScript Object Notation (JSON), ein kompaktes Datenformat in einer einfach lesbaren Textform für den Datenaustausch zwischen Anwendungen.

| |
|---|
| ```
{
"cameras": [
     {
       "id": "camera_1",
       "location": [-3.9, 3.1, 6.9]
     },
     {
       "id": "camera_2",
       "location": [5.8, 3.1, 0.01]
     }
   ],
   "lights": [
     {
       "id": 1,
       "type": "POINT",
       "location": [-4, 3.19, 3],
       "energy": 200.0
     },
     {
       "id": 2,
       "type": "AREA",
       "location": [5.9, 3.19, 6.9],
       "energy": 500.0
     }
   ],
   "room": {
     "dim": {
       "x": [-4,6], "y": [0, 3.2], "z": [0,7]},
     "material": {
       "id": "6819f000-4575-41 ec-809e-4ff5b366fb60"
     }
   },
   "objects": [
     [
       {
         "id": "20007117-7948-4e0b-8dba-25c0ca6d1c30",
         "name": "hospital_bed",
         "class": "transport-bed",
         "location": [3.4, 0.0, 2.5],
         "rotation": 35
       },
       {
         "id": "90dc80ce-782b-4d85-8562-891aca6b09f4",
         "name": "staff_1",
         "class": "person",
         "location": [-2.67, 0.0, 4.2],
         "rotation": 237
       },
       { "id": "3bdce5b5-07b8-484a-84c0-49e59d758283",
 "name":"staff_2",
         "class":"person",
         "location": [1.7, 0.0, -0.31],
         "rotation": 56
       }
     ],
     [
       {
         "id": "20007117-7948-4e0b-8dba-25c0ca6d1c30",
         "name":"hospital_bed",
         "class":"transport-bed",
         "location": [1.3, 0.0, 2.6],
         "rotation": 43
       },
       {
         "id": "90dc80ce-782b-4d85-8562-891aca6b09f4",
         "name":"staff_1",
         "class":"person",
         "location": [-2.1, 0.0, 1.67],
         "rotation": 211
       }
     ]
    ]
 }
``` |

Beispielcode einer Konfigurationsdatei "ample_scene_description.json"

In dem vorstehenden Beispielcode einer Konfigurationsdatei wird eine einfache Szene in einem Krankenhausraum mit zwei Personen und einem Krankenbett beschrieben.

Optional kann in einem weiteren Schritt 13 des erfindungsgemäßen Verfahrens eine Datenbank für Objekte oder Materialien, insbesondere für dreidimensionale Darstellungen beschriebene Objekte und/oder Materialien erzeugt werden. In dieser Datenbank können Beischreibungen und/oder Darstellungen von standardisierten Objekten und/oder Materialien hinterlegt werden, die durch entsprechende Bezugnahmen oder Verweise in der Konfigurationsdatei bei der Erzeugung der Bilddaten verwendet werden können.

Weiter in Bezugnahme auf Abbildung 1, kann in einem weiteren Schritt 14 des erfindungsgemäßen Verfahrens die Befehlsdatei auf den Prozessor zur Ausführung und zur Erzeugung eines Bilddatensatzes der zu simulierenden Szene übertragen werden. Die Ausführung der Befehlsdatei kann durch oder zusammen mit der Ausführung eines Computerprogramms zur Erstellung von CGI, wie beispielsweise Blender erfolgen. Die Übertragung der Befehlsdatei an den Prozessor kann zusammen mit der Konfigurationsdatei erfolgen oder diese kann alternativ beim Ausführen der Befehlsdatei eingelesen werden. Sofern in der Konfigurationsdatei auf vordefinierte, spezifizierte Objekte oder Materialien, insbesondere für dreidimensionale Darstellungen beschriebene Objekte und/oder Materialien verwiesen wurde, können in einem weiteren Schritt 16 des erfindungsgemäßen Verfahrens die entsprechenden, mit diesen Objekten und/oder Materialien assoziierten Daten aus der entsprechenden Datenbank abgerufen werden. Diese Daten können, wie vorstehend ausgeführt, Beschreibung, Bilddaten und/oder Kombinationen von Beschreibung und Bilddaten umfassen.

Auf dem Prozessor kann dann in einem weiteren Schritt 17 des erfindungsgemäßen Verfahrens, gegebenenfalls unter Ausführung eines Computerprogramms zur Erstellung von CGI, die mit der Konfigurationsdatei beschrieben Szene generiert werden und in Schritt 18 ein Bilddatensatz erstellt werden. Dieser Bilddatensatz ist eine Darstellung einer Szene gemäß der Konfigurationsdatei. Die einzelnen Bilder des Bilddatensatzes sind zweidimensionale Darstellungen der Szene zu einem jeweiligen Zeitpunkt aus dem Blickwinkel der in der Konfigurationsdatei beschriebenen Kamera. Bei zeitlichen Abläufen von Szenen werden Einzelbilder zu entsprechenden Zeitpunkten erzeugt, die, in zeitlicher Reihenfolge angeordnet einen entsprechenden Videostream darstellen. Sofern in der Konfigurationsdatei mehrere Kameras beschrieben sind, können verschiedene, dem jeweiligen Blickwinkel der Kamera entsprechende Bilddatensätze erzeugt werden. In diesem Fall können die einzelnen Bilddatensätze über entsprechende Zeitzuordnungen korreliert werden, das heißt, dass Abläufe einer Szene aus verschiedenen Kameraperspektiven erfasst werden können. Wenn der Simulation eine erweiterte Befehlsdatei mit assoziierten Objekten und/oder Materialien, die mit entsprechenden Bilddaten hinterlegt sind, zugrunde gelegt wird, werden diese Bilddaten in die Simulation der Szene integriert, und entsprechend für die Erstellung der Bilddatensätze verwendet. So kann beispielsweise, wenn eine Szene in einem real existierenden Operationssaal, der mit entsprechendem Bildmaterial hinterlegt ist, die Szene auf das Bildmaterial augmentiert werden. Auf diese Weise können hybride, das heißt, auf realen und auf gerenderten Daten bestehenden Bilddatensätze erstellt werden, wodurch, im Vergleich zu vollständig gerenderten Bilddatensätzen, die Heterogenität des Bildmaterials gesteigert werden kann und so der Wert als Trainingsdaten erhöht wird. Darüber hinaus können auch Filter angewendet werden, um dem gerenderten Bildmaterial eine realistischere Erscheinung zu geben. Insbesondere können die Filter, die, basierend auf in echten Bildern erkannten Kamera-Charakteristika erstellt wurden, auf gerendertes Bildmaterial angewandt werden, um so ein realistischeres, beziehungsweise natürlicheres Erscheinungsbild zu erzeugen.

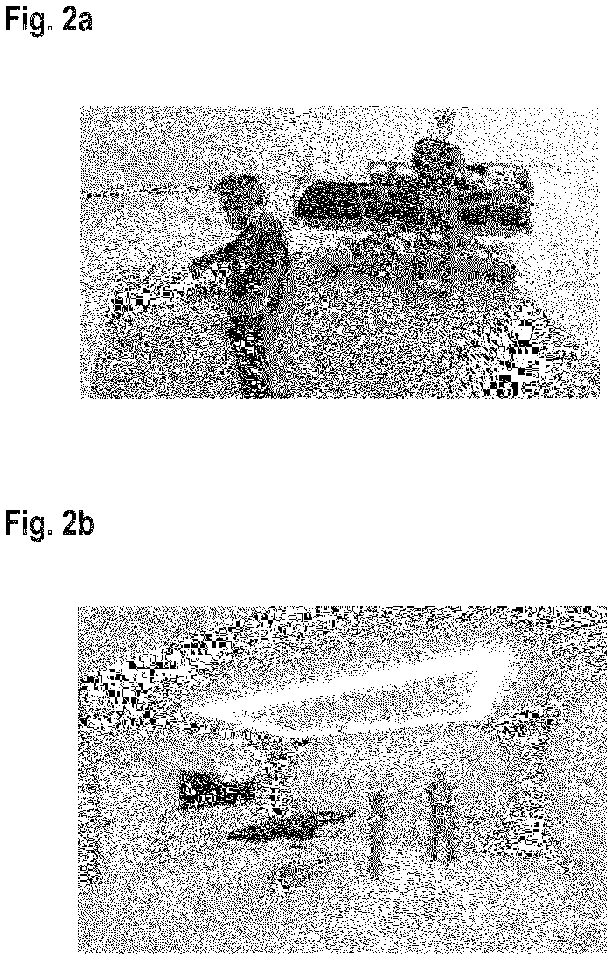

Abbildungen 2a und 2b zeigen exemplarisch Bilder zweier Szenen in verschiedenen Operationssälen. Abbildung 2a zeigt hierbei zwei Mitglieder des Krankenhauspersonals und ein leeres Krankenbett in einem Operationssaal, wobei das Krankenbett von einer der Personen teilweise verdeckt wird. Der Boden des Operationssaals ist hier zweifarbig ausgestaltet.

Abbilddung 2b zeigt einen Operationssaal mit zwei Mitgliedern des Krankenhauspersonals, einem leeren Operationstisch, zwei Operationslampen, einer rechteckigen Deckenbeleuchtung, einer Tür und einer rechteckigen, nicht beleuchteten und somit schwarzen Anzeigeeinheit an der Wand.

Weiter in Bezugnahme auf Abbildung 1, können in einem weiteren Schritt 19 des erfindungsgemäßen Verfahrens neben dem Erzeugen der Bilddatensätze auch mit den Bilddatensätzen assoziierte Annotationsdateien erzeugt werden. Dieser Vorgang kann, wie im vorstehenden Beispielcode des Befehlsdatei, ebenfalls in der Befehlsdatei (hier "reate_annotation(bpy, camera_id, type='bounding_box', file=annotation_file, format='CVAT for Images 1.1')") zur Ausführung definiert werden; in diesem Beispiel werden die Raumkoordinaten der Szene in der 'bpy.context' Instanz gespeichert. Diese Koordinaten zusammen mit der Kamera ermöglichen die Berechnung der 2D Bildkoordinaten für die Annotationsdatei. Entsprechend kann die Erstellung der Annotationsdatei zusammen mit der Erstellung der Bilddaten oder alternative nachfolgend unter Verwendung der Bilddaten erfolgen. Die Annotationsdateien können in verschiedenen Formaten, wie beispielsweise den im nachfolgenden Beispielcode verwendeten "VAT for Images" erstellt werden.

| |
|---|
| ```
 <?xml version="1.0" encoding="utf-8"?>
 <annotations>
  <version>1.1</version>
  <meta>
   <task>
    <id>76</id>
    <name>Blender Sample Scene</name>
    <size>1</size>
    <mode>annotation</mode>
    <overlap>0</overlap>
    <bugtracker></bugtracker>
    <created>2023-02-14 13:28:21.787704+00:00</created>
    < u pdated >2023-02-14 13:29:09.809934+00:00</updated>
    <subset>default</subset>
    <start_frame>0</start_frame>
    <stop_frame>0</stop_frame>
    <frame_filter></frame_filter>
    <segments>
     <segment>
      <id>394</id>
      <start>0</start>
      <stop>0</stop>
      <url>http://10.3.4.9/?id=394</url>
     </segment>
    </segments>
    <owner>
     <username>labeler</username>
     <email></email>
    </owner>
    <assignee></assignee>
    <labels>
     <label>
      <name>transport-bed</name>
      <color>#cc3366</color>
      <attributes>
      </attributes>
     </label>
     <label>
      <name>person</name>
      <color>#24b353</color>
      <attributes>
      </attributes>
     </label>
    </labels>
   </task>
   <dumped>2023-02-14 13:29:21.934285+00:00</dumped>
  </meta>
  <image id="0" name="or_sample_camera_1_1.jpg" width="1280" height="720">
   <box label="person" occluded="0" source="manual" xtl="653.29" ytl="114.57" xbr="719.27"
 ybr="292.83" z_order="0">
   </box>
   <box label="person" occluded="0" source="manual" xtl="446.09" ytl="140.03" xbr="531.75"
 ybr="406.27" z_order="0">
   </box>
   <box label="transport-bed" occluded="0" source="manual" xtl="267.83" ytl="328.71"
 xbr="770.21" ybr="718.81" z_order="0">
   </box>
  </image>
 </annotations>
``` |

Beispielcode einer Annotationsdatei "ample_annotations_cvat.xml"

Diese Annotationsdateien basieren im Wesentlichen auf den Beschreibungen in den jeweiligen Konfigurationsdateien. Die Kommentierung umfasst die Kategorisierung und das Labeln der Bilddatensätze damit diese als Trainingsdaten durch eine Maschine verarbeitet werden können, so dass die Bilddatensätze also maschinell erfassbar werden. Durch die Kommentierung werden entsprechend jedem Bild Informationen hinzugefügt, was auf dem jeweiligen Bild an welcher Stelle zu sehen ist. Da für jedes Objekt umfangreiche Informationen, wie Position, Dimension, usw. zur Verfügung stehen, können sehr präzise Kommentierungen erstellt werden. Anders als bei der Kommentierung von herkömmlichen Bilddaten ist, gemäß der vorliegenden Erfindung, Information über den gesamten Inhalt der Szene, also über die Umgebung, Parameter, Objekte usw. vorhanden. Entsprechend kann die Kommentierung automatisch in nahezu beliebig hoher Granularität erfolgen. Auch die Kategorisierung kann, basierend auf den Konfigurationsdateien, automatisch erfolgen, da der Inhalt der Szenen bekannt ist. Basierend auf der Befehlsdatei, beziehungsweise auf der erweiterten Befehlsdatei, kann für jedes Einzelbild eines Bilddatensatzes eine entsprechende Annotationsdatei erzeugt werden.

Wenn Variationen der Konfigurationsdateien erzeugt wurden, können auch für die daraus resultierenden Befehlsdateien und Bilddatensätze Annotationsdateien erzeugt werden. Die Bilddatensätze können in verschiedenen dem Fachmann bekannten Formaten, wie Yolo, COCO oder Pascal-VOC erzeugt werden. Die Bilddatensätze stellen zusammen mit den zugehörigen Annotationsdateien strukturierte Trainingsdatensätze dar.

Weiter in Bezugnahme auf Abbildung 1, können in einem weiteren Schritt 20 des erfindungsgemäßen Verfahrens bestimmte, vorzugsweise entsprechend vordefinierte Parameter der Konfigurationsdatei variiert werden. Entsprechend können beispielsweise Anordnung und/oder Bewegung bestimmter Objekte verändert werden und/oder die Beleuchtung kann verändert werden. Das erfindungsgemäße Verfahren ist so ausgestaltet, dass zufällige Veränderungen an der Konfigurationsdatei vorgenommen werden und basierend auf diesen veränderten Konfigurationsdateien weitere Bilddatensätze erstellt werden. Entsprechend können durch Variationen der Konfigurationsdateien, Bilddatensätze von ähnlichen Szenen gleichen Inhalts erzeugt werden. Die zufälligen Veränderungen der Konfigurationsdateien erfolgen vorzugsweise innerhalb der definierten, erlaubten und verbotenen Zustände. So können Bewegungen und/oder Aufenthaltsorte von Personen innerhalb der definierten Zustände variiert werden. Gleichermaßen kann auch die Bewegung von Objekten, beispielsweise von einem Krankenbett und/oder Operationslampen in einem Operationssaal innerhalb der definierten Zustände variiert werden. Durch diese Variationen können Bilddatensätze mit unterschiedlichen Überlagerungen, Beleuchtungs- und Schattierungseffekten und Positionen von Objekten erzeugt werden, wie sie auch in einer realen Szene mit demselben Inhalt und in derselben Umgebung vorkommen könnten. Grundsätzlich können durch den Benutzer die Elemente bestimmt werden, die variiert werden sollen. Vorzugsweise erfolgt die Variation jedoch im Rahmen der vordefinierten Zustände. Durch diesen Schritt des Verfahrens kann - basierend auf einer initialen Beschreibung einer Szene durch den Benutzer - automatisch die Diversität der Bilddatensätze und somit die Heterogenität der Trainingsdaten erhöht werden und auch der, gegebenenfalls aus der Beschreibung durch den Benutzer resultierende, menschliche Bias reduziert werden. Insgesamt kann somit die Eignung der erzeugten Bilddatensätze als Trainingsdaten gefördert werden.

Die mit dem erfindungsgemäßen Verfahren erzeugten Trainingsdatensätze können Vorhersagealgorithmen, wie Objekterkennungsalgorithmen für die Anwendung in ähnlichen Umgebungen oder in den realen Umgebungen, die in den Konfigurationsdateien definiert wurden, trainiert werden. Wenn Umgebungsspezifische Vorhersagealgorithmen erstellt werden, können diese für die spezifische Anwendung ausgewählt werden. So kann beispielsweise ein für einen bestimmten Operationssaal erzeugter Objekterkennungsalgorithmus zur Überwachung des entsprechenden Operationssaals ausgewählt werden. Alternativ, oder in Ergänzung dazu kann, über eine in dem Operationssaal angeordnete Raumkamera ein Bild des Raumes auswerten und so den jeweiligen Operationssaal erkennen und den zugehörigen, auf diesen Saal trainierten Algorithmus auswählen und für die Raumüberwachung verwenden. Gleichermaßen können auch spezifische Algorithmen aufgrund anderer Parameter, wie beispielsweise der Art eines medizinischen Eingriffs, entweder manuell oder automatisch, entweder über die vorhandenen Objekte und / oder Personen oder in Verbindung mit weiteren Informationen, wie einem Operationsplan, ermittelt werden. Durch die spezifisch trainierten Vorhersagealgorithmen können die Vorhersagen in dem jeweiligen zugehörigen Umfeld verbessert werden.

Basierend auf erfindungsgemäß erzeugte Bilddatensätze und Annotationsdateien können Objekterkennungsmodelle trainiert werden.

In einer Anwendung der erfindungsgemäßen Lehre wurden erzeugte Bilddatensätze zusammen mit Annotationsdateien für eine einfache OP-Szene mit 2 Mitarbeitern und einem Transportbett erstellt. Die Mitarbeiter und das Transportbett wurden automatisch im Raum bewegt. 500 Bilder wurden automatisch für zwei Raumkameras mit unterschiedlichen Blickwinkeln gemäß einer entsprechenden Befehlsdatei automatisch gerendert, wobei auch Bilder erzeugt wurden, in denen das Transportbett ganz oder teilweise durch die Mitarbeiter verdeckt wurde. Ein Beispiel eines dieser Bilder ist in Abbildung 2Xa gezeigt. Da sämtliche Objekte und Bewegungen durch die Befehlsdatei, beziehungsweise die Konfigurationsdatei, bekannt waren, konnten detaillierte Annotationsdateien erstellt werden. Mit den erzeugten Daten konnte ein Objekterkennungsmodell (YoloX), dass vorher mit einem beschränkten Trainingsdatensatz basierend auf echtem Bildmaterial trainiert wurde, in seiner Detektionsgenauigkeit erheblich gesteigert werden.

Der Umfang dieser Offenbarung schließt alle Änderungen, Ersetzungen, Variationen, Abwandlungen und Modifikationen an den hierin beschriebenen oder veranschaulichten Ausführungsbeispielen ein, die ein durchschnittlicher Fachmann begreifen würde. Der Schutzumfang dieser Offenbarung ist nicht auf die hierin beschriebenen oder veranschaulichten Ausführungsbeispiele beschränkt. Obwohl diese Offenbarung die jeweiligen Ausführungsformen hierin als bestimmte Komponenten, Elemente, Merkmale, Funktionen, Operationen oder Schritte umfassend beschreibt und veranschaulicht, können zudem beliebige dieser Ausführungsformen beliebige Kombinationen oder Permutationen beliebiger Komponenten, Elemente, Merkmale, Funktionen, Operationen oder Schritte, die irgendwo hierin beschrieben oder veranschaulicht sind, umfassen, die ein durchschnittlicher Fachmann begreifen würde. Ein Verweis in den angehängten Patentansprüchen darauf, dass ein Verfahren oder eine Vorrichtung oder eine Komponente einer Vorrichtung oder ein System zum Durchführen einer bestimmten Funktion angepasst, eingerichtet, fähig, konfiguriert, befähigt, betriebsfähig oder betriebsbereit ist, schließt des Weiteren dieses Gerät, dieses System oder diese Komponente ein, unabhängig davon, ob es/sie oder diese bestimmte Funktion aktiviert, eingeschaltet oder freigegeben ist, solange dieses Gerät, dieses System oder diese Komponente dazu angepasst, eingerichtet, fähig, konfiguriert, befähigt, betriebsfähig oder betriebsbereit ist. Obwohl diese Offenbarung bestimmte Ausführungsformen als bestimmte Vorteile bereitstellend beschreibt oder veranschaulicht, können zudem bestimmte Ausführungsformen keine, einige oder alle diese Vorteile bereitstellen.

## Patentansprüche

1. Computerimplementiertes Verfahren zum Erzeugen anwendungsspezifischer Bilddaten zum Trainieren einer auf künstlicher Intelligenz basierender Objekterkennung in Bildern medizinischer und/oder klinischer Arbeitsabläufe umfassend:
Erzeugen einer Befehlsdatei zum Ausführen auf einem Prozessor zur Erzeugung eines bis zu dreidimensionalen Bilddatensatzes unter Verwendung wenigstens einer Konfigurationsdatei;
Erzeugen wenigstens einer Konfigurationsdatei die eine zu simulierende Szene beschreibt, wobei die Konfiguration wenigstens eine Kamera und wenigstens eine Lichtquelle beschreibt;
Übertragen und Ausführen der Befehlsdatei und der Konfigurationsdatei auf den Prozessor zum Erzeugen des Bilddatensatzes, wobei der Bilddatensatz perspektivisch der wenigstens einen Kamera entspricht und die wenigstens eine Lichtquelle berücksichtigt;
Erzeugen einer mit dem Bilddatensatz assoziierten Annotationsdatei; und
Speichern des Bilddatensatzes zusammen mit der Annotationsdatei.

2. Verfahren nach Anspruch 1, wobei die Konfigurationsdatei eine statische oder dynamische Szene beschreibt.

3. Verfahren nach Anspruch 1 oder 2, wobei die Konfigurationsdatei allgemeine Parameter, Objekte, Materialien, Beleuchtung, Kameraeigenschaften, Objektpositionen, Objektbewegungen Umgebungseigenschaften, Okklusionsebenen und/oder zeitabhängige Änderungen von Parametern umfasst.

4. Verfahren nach Anspruch 3, wobei die Konfigurationsdatei auf Objekten und/oder Materialien in einem nicht flüchtigen Speicher verweist die bei dem Ausführen der Befehlsdatei zum Erzeugen des Bilddatensatzes hinzugefügt werden.

5. Verfahren nach Anspruch einem der vorstehenden Ansprüche, wobei die in der Konfigurationsdatei beschriebene Szene in einer vorbestimmten Umgebung zu simulieren ist, wobei die vorbestimmte Umgebung einen realen Raum beschreibt.

6. Verfahren nach Anspruch 5, wobei die Beschreibung des realen Raums über eine Scanvorrichtung erfasst wird und/oder digitale Bilder des Raums umfasst.

7. Verfahren nach einem der Ansprüche 3 bis 6, wobei die allgemeinen Parameter eine Beschreibung der zu simulierenden Szene umfassen.

8. Verfahren nach einem der vorstehenden Ansprüche, wobei basierend auf Variationen der in der Konfigurationsdatei enthaltenen Beschreibungen weitere Bilddatensätze und mit den weiteren Bilddatensätzen assoziierte Annotationsdateien erzeugt werden.

9. Verfahren nach Anspruch 8, wobei die Konfigurationsdatei erlaubte und verbotene Zustände der Objekte und der Beleuchtung enthält und die Variationen durch diese Zustände definiert werden.

10. Verfahren nach Anspruch 8, wobei die Variationen die wenigstens eine Lichtquelle betreffen.

11. Verfahren nach einem der vorstehenden Ansprüche, wobei die erzeugten Bilddatensätze, und die mit ihnen assoziierten Annotationsdateien, kategorisiert gespeichert werden.

12. Verfahren nach einem der vorstehenden Ansprüche, wobei die Konfigurationsdatei wenigstens teilweise Informationen basierend auf einer Voreinstellung enthält.

13. Verfahren zum Erzeugen einer anwendungsspezifischen, auf künstlicher Intelligenz basierender Objekterkennung, umfassend das Trainieren eines Vorhersagealgorithmus mit nach einem der Ansprüche 1 bis 12 erstellten Trainingsdaten.

14. Verfahren zum Erkennen von Objekten in einer spezifischen Anwendung, wobei ein mit nach einem der Ansprüche 1 bis 12 erstellten Trainingsdaten trainierter Vorhersagealgorithmus auf wenigstens ein digitales Bild angewendet wird und wobei das wenigstens eine Bild, ein Bild einer Szene ist, die Grundlage der erstellten Trainingsdaten ist.

15. Verfahren nach Anspruch 14, wobei der Vorhersagealgorithmus nach Erkennung einer Szene die Grundlage der entsprechenden erstellten Trainingsdaten ist ausgewählt wird.
